Europäisches Patentamt

European Patent Office

Office européen des brevets

(19)

(11) Publication number: **0 468 182 A1**

# EUROPEAN PATENT APPLICATION

(12)

(21) Application number: **91109497.7**

(22) Date of filing: **10.06.91**

(51) Int. Cl.5: **A61K 37/30**, A61K 9/06, A61K 9/72, A61K 47/26

(30) Priority: **12.06.90 IT 2061290**

(43) Date of publication of application:
**29.01.92 Bulletin 92/05**

(84) Designated Contracting States:
**AT BE CH DE DK ES FR GB GR IT LI LU NL SE**

(71) Applicant: **SCLAVO S.p.A.**
**Via Fiorentina 1**
**I-53100 Siena(IT)**

(72) Inventor: **Lattanzi, Filippo**
**Via Sansedoni, 9**

**I-53100 Siena(IT)**
Inventor: **Ceschel, Giancarlo**
**Piazza 6 Febbraio, 14**
**I-20145 Milan(IT)**
Inventor: **Vanni, Riccardo**
**Via Montesanto, 3**
**I-53100 Siena(IT)**

(74) Representative: **Gervasi, Gemma et al**
**NOTARBARTOLO & GERVASI Srl Viale**
**Bianca Maria 33**
**I-20122 Milan(IT)**

(54) Pharmaceutical composition in powder form for nasal administration containing essentially calcitonin and a water-soluble excipient.

(57) A pharmaceutical composition in powder form for nasal administration is described, comprising essentially a calcitonin and a water-soluble excipient.

FIG. 1

Field of the invention

This invention relates to a pharmaceutical composition in powder form for nasal administration comprising a calcitonin and a water-soluble excipient as essential components.

State of the art

Calcitonins are calcium-regulating hormones which are secreted by the thyroid gland in mammals and by the ultimo-branchial gland in non-mammals.

They have a polypeptide structure in the form of a single chain containing 32 amino acids. However, the amino acid sequence varies substantially according to the hormone source, mammal calcitonins (substantially human and pig calcitonin) differing substantially from non-mammal calcitonins (substantially salmon and eel calcitonin), these latter showing greater specific biological activity (I.U./mg).

The substantial action of calcitonins is to oppose the effects of the parathyroid hormone at the bone and renal level by inhibiting bone resorption and manifesting a hypocalcemic and hypophosphatemic action. Administration of animal calcitonin is therefore used in the treatment of acute hypercalcemia associated with neoplastic forms, hyperparathyroidism and intoxication by vitamin D. It is also used in the treatment of infantile idiopathic hypercalcemia, in osteoporosis, in Sudeck's disease and in Paget's disease.

Calcitonin is generally administered parenterally, and in particular subcutaneously or intramuscularly.

Formulations for administering calcitonin via the nasal mucosa are also known. European patent application 84301546.2 describes pharmaceutical compositions in powder form for nasal administration comprising the active principle and a support base able to absorb water but insoluble in it, its purpose being to ensure that the active principle remains on the nasal mucosa for a time sufficient for its absorption. This approach has however the drawback of using an extraneous body as represented by said support base.

Pharmaceutical compositions for polypeptide nasal administration which also comprise absorption promoters are described in European patent EP-A-302,772. Again however, for its effectiveness the composition requires the use of absorption additives, and these can exert an irritating effect on the mucosa.

Detailed description of the invention

According to the present invention, it has now been surprisingly found that a pharmaceutically acceptable calcitonin composition in powder form can be formulated which is suitable for nasal administration without the simultaneous presence of special absorption agents or aids, but simply by mixing calcitonin with a water-soluble excipient, even though this latter component is totally free of those properties which were considered essential in previously described compositions (incorporation of an absorption additive or an unabsorbable extraneous phase for transporting the medicament).

The composition obtained in this manner is reliable, stable, does not irritate the nasal mucosa and does not reduce blinking frequency.

The present invention therefore relates to a pharmaceutical composition in powder form for nasal administration, comprising:
-   a calcitonin
-   a water-soluble excipient.

The active principle usable in the formulation of the pharmaceutical composition according to the present invention can be chosen from calcitonins of natural or synthetic origin, such as salmon calcitonin (SCT), eel calcitonin (ECT), pig calcitonin or the synthetic analogues such as $[ASU^{1-7}]ECT$ (commonly known as carbocalcitonin) etc.

The calcitonins usable according to the invention can be in free form or in the form of a pharmaceutically acceptable salt or complex, for example in the form of a salt of addition with a pharmaceutically acceptable acid. These salts or complexes are known and possess a degree of activity and tolerance equivalent to those of the free form. The salts of addition with acids appropriate for use in the invention comprise, for example, the hydrochlorides and acetates.

The quantity of active principle present in the pharmaceutical compositions of the invention can vary from 50 to 500 International Units (I.U.) of calcitonin.

Water-soluble excipients include for example mannitol, lactose, fructose, glucose, albumin etc.

The quantity of water-soluble excipient is between 5 and 50 mg. The formulation is prepared by mixing the active principle with suitably dried mannitol.

Both the active principle and the mannitol are reduced to the same particle size (50-100 $\mu$m), preferably

50-60 μm.

After mixing the powders together and checking that uniform mixing has been obtained by taking samples, the powder is placed in a dispensing-encapsulating machine which distributes in into hard gelatin capsules of suitable capacity.

At the moment of use the capsules are inserted into a suitable device which perforates them to enable the powder to be inhaled or aspired.

Any electrical or mechanical device can be used which makes the entire contents of the capsule available for inhalation or aspiration.

The following example non-limitingly illustrates the preparation of certain formulations and unit dosage forms of the present invention.

EXAMPLE

Exactly weighed quantities of calcitonin and mannitol are intimately mixed together in a suitably sized mixer preferably with movement about an eccentric axis to provide more complete and rapid mixing.

A sample is taken occasionally to check the uniformity of the mixtures. When mixing is complete the powder is distributed into hard gelatin capsules of suitable size, preferably using the insertion-closure type. All the capsules are then cleaned of powder and inserted into a PVC-aluminium blister pack or into a strip of aluminium for packaging.

Hard gelatin capsules were prepared each containing:

| - eel calcitonin (ECT) | 100 I.U. |
|---|---|
| - mannitol | 20 mg. |

Other formulations of the invention, obtained by an analogous procedure, consist of:

1)
ECT          100 I.U.
Mannitol     20 mg
2)
ECT          150 I.U.
Mannitol     20 mg
3)
SCT          50 I.U.
Mannitol     20 mg
4)
SCT          100 I.U.
Mannitol     20 mg
5)
SCT          150 I.U.
Mannitol     20 mg
6)
$ASU^{1-7}ECT$     50 I.U.
Mannitol          20 mg
7)
$ASU^{1-7}ECT$     100 I.U.
Mannitol          20 mg
8)
$ASU^{1-7}ECT$     150 I.U.
Mannitol          20 mg

The capsules obtained in this manner were inserted into a suitable device which by perforating the capsules enables the contents to be quantitatively extracted, either by aspiration or by insufflation.

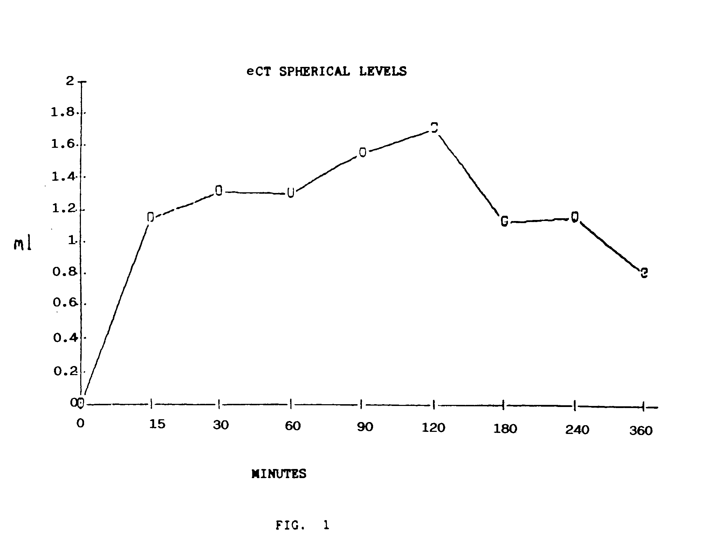

The medicament (one 100 I.U. capsule) was administered nasally to 5 healthy volunteers, from whom blood samples were taken at 0, 15, 30, 60, 90, 120, 180, 240 and 360 minutes. The calcitonin concentration in the serum was determined by the radio-immunological assay (RIA) method. The results are shown in Figure 1 in which the vertical axis represents the mean plasmatic Ca levels and the horizontal axis represents minutes.

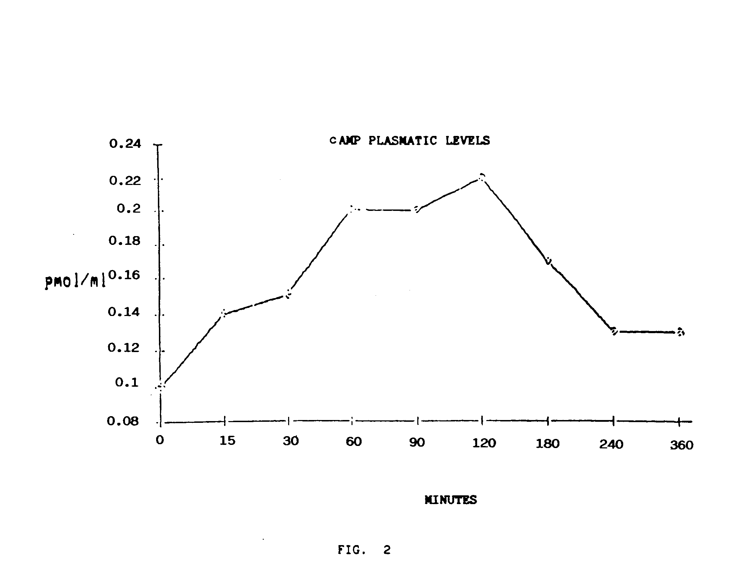

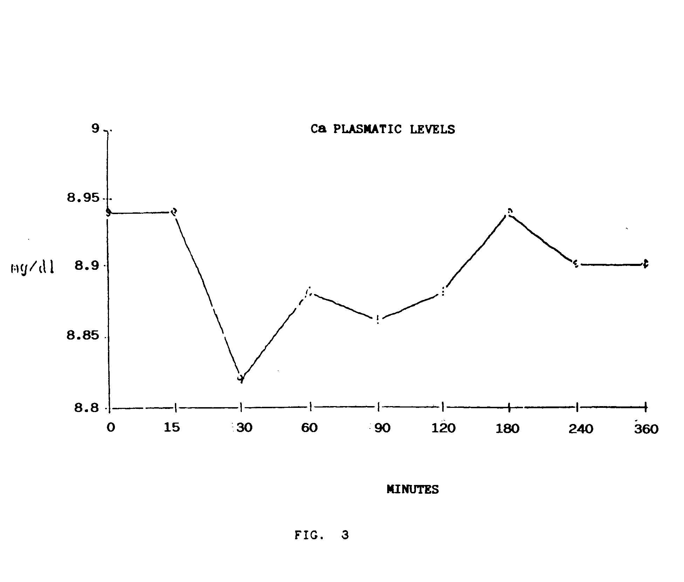

Figures 2 and 3 show the mean cAMP and hematic calcium levels respectively. These data shown the

EP 0 468 182 A1

excellent bioavailability of calcitonin when administered by the compositions according to the invention. No side effects were noted following administration of the new compositions.

## Claims

1. A pharmaceutical composition in powder form for nasal administration comprising a calcitonin and a water-soluble excipient as essential components.

2. A pharmaceutical composition as claimed in claim 1, wherein the calcitonin is eel calcitonin.

3. A pharmaceutical composition as claimed in claim 1, wherein the calcitonin is salmon calcitonin.

4. A pharmaceutical composition as claimed in claim 1, wherein the calcitonin is pig calcitonin.

5. A pharmaceutical composition as claimed in claim 1, wherein the calcitonin is $ASU^{1-7}ECT$.

6. A composition as claimed in claims 1 to 5, wherein the water-soluble excipient is mannitol, lactose, fructose, glucose or albumin.

7. A composition as claimed in claim 6, wherein the water-soluble excipient is mannitol.

8. A pharmaceutical composition as claimed in claims 1 to 7, wherein the active principle quantity is between 50 and 500 I.U.

9. A pharmaceutical composition as claimed in claim 8, wherein the water-soluble excipient quantity is between 5 and 50 mg.

10. A composition as claimed in claim 9, wherein the particle size of the components is between 50 and 100 $\mu$m.

11. A composition as claimed in claim 10, wherein the particle size of the components is between 50 and 60 $\mu$m.

12. The use of a composition claimed in claims 1 to 11 in the preparation of pharmaceutical compositions for the treatment of hypercalcemia, osteoporosis, Sudeck's disease and Paget's disease.

13. Be use of the compositions claimed in claims 1 to 11 for treating hypercalcemia, osteoporosis, Sudeck's disease and Paget's disease.

4

FIG. 1

FIG. 2

Ca PLASMATIC LEVELS

FIG. 3

European Patent Office

# PARTIAL EUROPEAN SEARCH REPORT

which under Rule 45 of the European Patent Convention shall be considered, for the purposes of subsequent proceedings, as the European search report

Application number

EP 91 10 9497

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | PATENT ABSTRACTS OF JAPAN, vol. 10, no. 314, (C-380)(2370), 24th October 1986; & JP-A-61 126 034 (YAMANOUCHI PHARMACEUT. CO. LTD) 13-06-1986 <br><br> * The whole abstract * <br><br> -- |  1-6,12 | A 61 K 37/30 <br> A 61 K 9/06 <br> A 61 K 9/72 <br> A 61 K 47/26 |
| X | EP-A-0 364 235 (TOYO JOZO K.K.) <br><br> * Abstract; page 2, line 55 - page 3, lines 4; line 4, page 3, lines 24-31; page 3, line 51 - page 4, line 5; examples 1-3; claims * <br><br> -- | 1-8, 10,12 |  |
| X | FR-A-2 623 090 (I.S.F. S.p.A.) <br><br> * Abstract; page 10, line 32 - page 11, line 4; examples 49-50 * <br><br> -- <br> ./. | 1-6,8, 9,12 | TECHNICAL FIELDS SEARCHED (Int. Cl.4) <br><br> A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-12

Claims searched incompletely:

Claims not searched: 13

Reason for the limitation of the search:

Method for treatment of the human or animal body by surgery or therapy (see Art. 52(4) of the European Patent Convention).

| Place of search | Date of completion of the search | Examiner |
|---|---|---|
| THE HAGUE | 31-07-1991 | HOFF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| X | WO-A-89 03 207 (COSMAS-DAMIAN LTD)<br><br>* Abstract; page 5, lines 4-18; page 7 line 22 - page 8, line 1; page 11, line 17 - page 12, line 7; page 10, lines 1-6; claims 1-6, 9,11-15 *<br><br>-- | 1-6, 10-12 | |
| D,X | EP-A-0 302 772 (TOYO JOZO CO. LTD)<br><br>* Abstract; page 3, lines 8-12; examples 2-6; claims *<br><br>-- | 1-8,10, 12 | TECHNICAL FIELDS SEARCHED (Int Cl 4) |
| A | EP-A-0 308 181 (NOVO INDUSTRI A/S)<br><br>* Abstract; column 3, lines 7-13; column 4, lines 1-9,18-45; column 5, line 65 - column 6, line 11; claims *<br><br>-- | 1-12 | |
| P,A | EP-A-0 417 930 (SUMITOMO PHARMACEUTICALS CO. LTD)<br><br>* Abstract; examples 2,5; claims * | 1-12 | |

----

EPO Form 1505.3   06.78